# EUROPEAN PATENT SPECIFICATION

(11) **EP 3 946 145 B1**
(45) Date of publication and mention of the grant of the patent: **26.07.2023**
(21) Application number: 20739270.5
(22) Date of filing: 09.06.2020
(51) Int. Cl.: A61F 2/966

(54) **A CATHETER DEVICE FOR TRANSLUMINALLY DELIVERING A SELF-EXPANDING TUBULAR IMPLANT TO A SITE IN A BODY**
KATHETERVORRICHTUNG ZUR TRANSLUMINALEN ABGABE EINES SELBSTEXPANDIERENDEN ROHRFÖRMIGEN IMPLANTATS AN EINE STELLE IN EINEM KÖRPER
DISPOSITIF DE CATHÉTER POUR ADMINISTRATION TRANSLUMINALE D'UN IMPLANT TUBULAIRE AUTO-EXPANSIBLE À UN SITE CORPOREL

(43) Date of publication of application: 09.02.2022
(73) Proprietor: Angiomed GmbH & Co. Medizintechnik KG, 76227 Karlsruhe (DE)
(72) Inventor: GOEBBELER, Peter, 76227 Karlsruhe (DE)
(74) Representative: Hoffmann Eitle
(86) International application number: PCT/EP2020/065920
(87) International publication number: WO 2021/249622

(56) References cited:
- US-A1- 2004 204 749
- US-A1- 2004 267 348
- US-A1- 2007 156 223
- US-A1- 2009 171 427

## Description

### TECHNICAL FIELD

The present invention relates to a catheter device for transluminally delivering a self-expanding tubular implant to a site in a body, a method of making a shape memory alloy tube for a catheter device for transluminally delivering a self-expanding tubular implant to a site in a body and a method of assembling a catheter assembly for transluminally delivering a self-expanding tubular implant to a site in a body.

### BACKGROUND

Catheter devices are known. Catheter devices may comprise an inner catheter and a sheath. An implant for delivery to a site in the body is loaded onto the inner catheter. To deliver the stent, the sheath is pulled back. The implant may be compressed on the inner catheter.

In such conventional catheter devices, it has been found that it is difficult to maintain a firm grip of the compressed implant on the inner catheter during retraction of the sheath. In particular, it has been found that this is the case if the implant or a covering on the implant weight varies, as is generally the case due to material and process tolerances. Implants that are too large or heavy may lead to too much compression and the associated deployment forces may exceed the strength limit of the delivery system joints, leading to delivery system failure. Further, it has been found that implants that are relatively small or lightweight may lose grip on the inner catheter and therefore, may be retracted along with the sheath, constituting a complete failure to deploy or significantly worse, a partially deployed implant.

In US 2010/0274226 A1 fingers are provided on the inner catheter. When the sheath is retracted the implant can be damaged as it slips over the inner catheter or the implant may compress axially and seize within the sheath such that deployment becomes impossible.

To address this problem, it is known to provide an elastic silicone sleeve on the inner catheter over which a section of a fine metal wire braid is placed to improve the grip.

The silicone sleeve is intended to give radial flexibility and accommodate tolerance related changes in the dimension and/or covering weight of the implant without a great deal of change in the radial compression of the catheter device (assembly) and hence without a great deal of change in the deployment force.

A disadvantage of this arrangement is that while silicone is a very soft and flexible material, i.e. well suited to conform, the wall thickness of the sleeve is very thin due to space constraints and the mechanical behavior of silicone can be described as linearly elastic. Therefore, increasing compression leads to increasing push-back, even though at a slow rate compared to stiffer materials, and thus requires a higher deployment force.

US 2004/0204749 A1 is concerned with a stent delivery system with securement and deployment accuracy.

### SUMMARY OF THE INVENTION

It is an object of the invention to provide a catheter device that will address at least one of the above-mentioned problems.

In accordance with one aspect of the invention, there is provided a catheter device as defined in claim 1.

In this way, the plurality of retention features improves implant retention on the inner catheter over a wider range of implant weight and implant and catheter device component dimensions.

According to this aspect of the invention, at least one of the first plurality of implant retention elements is a flap arranged to extend distally from a root line in the cylindrical distal end component. In this way, the implant is prevented from moving in a proximal direction.

In one embodiment, the design of the plurality of retention elements is such that a hinge portion is in the plateau area (region) of the stress-strain curve of the material of the plurality of retention elements when engaged with the implant, so that the retention elements exert more or less the same force on the implant regardless of how much they protrude (deflect) from the cylinder, so it is not critical how much they deflect.

The properties of the plurality of retention elements ensures relatively constant friction and retention force by unidirectional elements, by virtue of a first plurality of implant retention elements, or bidirectional elements, by virtue of a first and a second plurality of implant retention elements, on the outside of the inner catheter. On account of the plateau area in the stress-strain curve of the material of the plurality of retention elements, these elements can accommodate varying degrees of radial displacement whilst exerting a constant radial force regardless of the degree to which the plurality of retention elements protrude from the inner catheter. In this way a relatively constant radial stress condition is ensured. Thus, relatively constant friction and deployment forces over a range of implant weight and implant and delivery system component dimensions are achieved. Thus, enhancing deployment reliability.

In a further embodiment, the cylindrical distal end component includes a second plurality of implant retention elements, which are configured to utilise a shape memory effect to protrude radially outward from the cylindrical distal end component to engage a radially inward facing surface of the implant to restrain the implant from being carried distally, relative to the cylindrical distal end component. In this way, the implant is prevented from moving in a distal direction. With both distal and proximal flaps, the implant can be prevented from moving in both directions.

Hereinbelow, in a distal flap (a first retention element), the flap is arranged to extend distally from a root line in the cylindrical distal end component. In this way, the implant is prevented from moving in a proximal direction. Accordingly, in a proximal flap (a second retention element), the flap is arranged to extend proximally from a root line in the cylindrical distal end component. In this way, the implant is prevented from moving in a distal direction.

In a further embodiment, the cylindrical distal end component is made of nitinol. In this way, the reliability of the deployment of the implant is enhanced, since nitinol has a rather flat plateau area in its stress-strain curve, that is, it can accommodate strain changes with relatively little stress changes. This material characteristic of, in particular, nitinol, results in a first and/or second plurality of retention elements that can accommodate diameter changes with relatively small radial force changes, making the degree of compression forces of the implant less dependent on the degree of spatial compression of the implant. Furthermore, the first and/or second plurality of retention elements, in one embodiment, are incorporated in the wall of the nitinol inner catheter member, rather than added on top of an existing inner catheter material. In this way, space is saved and can therefore, be used to accommodate the implant. In other words, more space is available in the catheter device for the implant.

In a yet further embodiment, at least one flexible section comprises a plurality of staggered circumferentially extending slits to form a cardanic joint. In this way, the slits endow the component with enhanced bending ability, thereby facilitating advance of the distal end of the device along especially tortuous lumens to hard to reach implant sites in the body.

In a yet further embodiment, at least the first plurality of retention elements and the second plurality of retention elements are disposed in the same implant retention section. In this way, the reliability to deploy short implants is enhanced.

In a further embodiment, the first and second plurality of retention elements are arranged circumferentially around the cylindrical distal end component in at least one row, wherein the first and second plurality of retention elements alternate along at least one row. In this way, the reliability to deploy short implants is further enhanced, since this arrangement allows for adjustment of the location of the implant (relative to the sheath, not relative to the inner catheter member) in both proximal and distal directions.

In a further embodiment, the first plurality of retention elements is disposed at a location on the cylindrical distal end component that is arranged to be received by a distal region of the implant.

In a yet further embodiment, the second plurality of retention elements are disposed at a location on the cylindrical distal end component that is arranged to be received by a proximal region of the implant.

In this way, the reliability to deploy long implants is enhanced, since, whichever way the implant is moved by pushing or pulling the inner catheter during implant (stent) position adjustment, it is ensured that the implant is pulled, rather than pushed, thus preventing buckling and seizing within the outer sheath.

A further advantage of the present invention is that it addresses a problem associated with deploying long and flexible self-expanding implants, such as vascular implants, such as nitinol stents or expanded polytetrafluoroehthylene (ePTFE) covered nitinol stents from a pull-back outer sheath type delivery system.

In an embodiment of the invention, the first and/or second plurality of retention elements are configured so that a force exerted on the implant by the first and/or second retention elements, respectively, within a range of deflection of the hinge is substantially the same over the range of deflection. In this way, regardless of the deflection, the force exerted on the implant is the same, therefore tolerances are not so critical. Further, the space required for the first and/or second retention elements is minimized in the radial direction, to thus minimize the radial dimensions of the catheter device.

In a further embodiment, the first plurality of implant retention elements are able to lie within the wall thickness. In this way, the and/or second retention elements require no space of their own, therefore, the diameter of the catheter device is yet further reduced.

According to a further aspect of the invention, there is provided a method of making a shape memory alloy tube for a catheter device for transluminally delivering a self-expanding tubular implant to a site in a body as defined in claim 22.

According to a yet further aspect of the invention, there is provided a method of assembling a catheter assembly for transluminally delivering a self-expanding tubular implant to a site in a body as defined in claim 23.

### BRIEF DESCRIPTION OF THE DRAWINGS

The invention, and various embodiments thereof, will further be explained on the basis of examples, with reference made to the drawings, in which:
Fig. 1 shows a catheter device and an implant according to an embodiment of the invention;
Fig. 2a shows a portion of an inner catheter in a relaxed position according to an embodiment of the invention;
Fig. 2b shows the portion of the inner catheter of Fig. 2a in a compressed position according to an embodiment of the invention;
Fig. 2c is a graph showing a stress-strain curve of the portion of the inner catheter shown in Figs. 2a and 2b;
Fig. 3 shows an inner catheter according to an embodiment of the invention;
Fig. 4 shows various embodiments and various views of the various embodiments showing a variety of retention features according to the various embodiments of the invention;
Fig. 5 shows various further embodiments and various views of the various embodiments showing a variety of retention features according to the various embodiments of the invention;
Fig. 6 shows an inner catheter according to further embodiments of the present invention;
Fig. 7 shows a retention feature according to an embodiment of the present invention and a method of making a shape memory alloy tube according to an embodiment of the present invention.

### DETAILED DESCRIPTION

In the drawings and in the detailed description hereinbelow, like reference signs denote like features. The invention is exemplified in the embodiments described below. The invention is not limited to these embodiments, which are schematically shown.

Hereinbelow, reference is made to the terms "proximal" and "distal". Where reference is made to a "proximal" component or part of a component, it is to be understood to refer to a component or part of a component that is disposed at a location towards a location of the device handle. Whereas, where reference is made to a "distal" component or part of a component, it is to be understood to refer to a component or part of a component that is disposed away (remote) from a location of the device handle.

When deploying a self-expanding implant, for example, a nitinol implant such as a stent or a ePTFE covered stent graft, from its compressed state inside a sheath of a pull-back type delivery system, the friction force generated between the retracting outer sheath and the implant, pointing to proximal (i.e. in the direction of the device handle) is typically balanced by axial compression forces in the inner catheter 2. The inner catheter 2 leads all the way to the device handle and typically also serves as a lumen to accommodate a guide wire over which the delivery system is tracked from an entry point into the body to the implant target site in the body, often through arteries or veins, and also, depending on the target site through other body ducts.

Conventionally, transferring the friction force from the implant 20 to the inner catheter 2 may be achieved by a collar on the inner catheter that is placed proximal to the implant. When the sheath is retracted the implant is moved back against the collar and the force is transmitted from the proximal implant end to the collar, and thus the inner catheter, and thus back to the delivery system handle. The inventor has found that when attempting to deploy, in particular, long and flexible implants, the implant tends to buckle under the axial compression and seize within the sheath, preventing successful deployment. Moreover, transmission of the entire implant sheath friction force through the proximal end of an ePTFE covered implant may lead to damage of the delicate ePTFE covering if the covering extends beyond the metal frame of the covered implant.

It has been found that a more reliable deployment is achieved with an arrangement whereby the transmission of the force from the implant to the inner catheter is through a part or parts of the entire interface between the inside of the compressed implant and the outside of the inner catheter. It has been found that in order to achieve this, based purely on friction, a significantly relatively higher coefficient of friction is required between the inside of the implant and the outer side of inner catheter than the coefficient of friction between the outside of the compressed implant and the inside of the sheath. In practice, every effort is made to keep friction as low as possible on the outside of the implant. It has been found, however, that it is difficult to achieve a high enough differential between a relatively low coefficient of friction on the "implant outer surface to a sheath" interface and a relatively high coefficient of friction on the "implant inner surface to the inner catheter" interface, in order to securely prevent any motion of the implant relative to the inner catheter when the sheath is retracted. It has been found that grip on the inner catheter can be augmented by appropriately structuring the surface of the inner catheter such that shape lock occurs between features on the inside of the implant, such as bare stent strut edges or compressed covering folds, and the structures on the outer surface of the inner catheter.

However, since the level of force required to retract the sheath is not only a function of the coefficient of friction between the implant and the sheath, but is also a function of the level of radial force between the implant and the sheath, it is desired to keep radial force as low as possible. This may be achieved, for example, by minimizing the degree of compression of the implant, which can be achieved by maximizing the space for accommodating the implant in the device. On the other hand, a minimum level of radial compression of the implant is required to ensure there is sufficient friction between the implant and the inner catheter. This degree of implant compression has to be kept within a defined range and should also take into account dimensional tolerances of the components involved, variations in the dimensions of the implant and the covering weight or dimensional changes of the implant and covering weight or dimensional changes of the implant and delivery system components caused by aging, changes in temperature and humidity or outside mechanical forces caused by device handling, such as during removal from the packaging, prepping and tracking through tortuous anatomy.

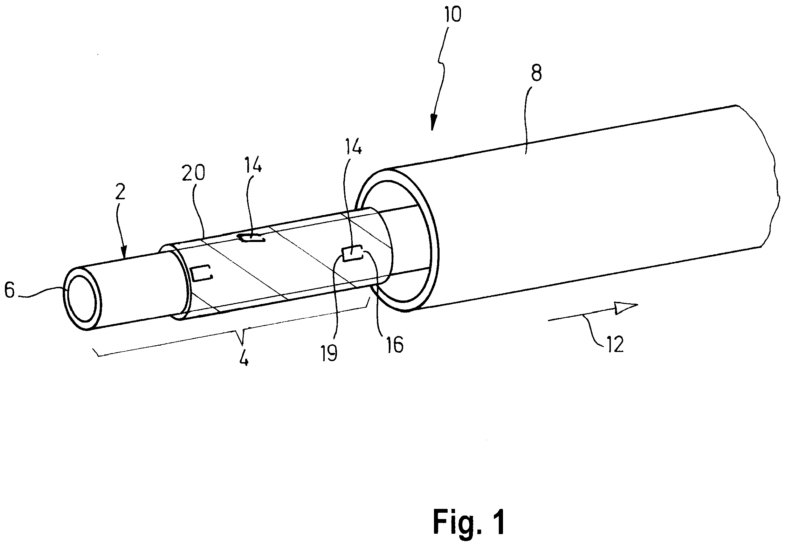

Fig. 1 shows a catheter device 10 and an implant 20 according to an embodiment of the invention. In the embodiment the catheter device is used to transluminally deliver a self-expanding tubular implant 20 to a site in a body. The implant may be a vascular implant, such as a stent. The implant 20 may be covered in a covering such as ePTFE or an uncovered implant, such as a nitinol stent. The catheter device of the embodiment may be used in a pull-back outer sheath type delivery system. Fig. 1 shows a pull-back delivery system. The catheter device 10 comprises an inner catheter 2. The inner catheter 2 may comprise a cylindrical distal end component 4 with a wall 6. The cylindrical distal end component 4 may be arranged, in use, to be received within a lumen of the implant 20. The catheter device 10 may further comprise a sheath 8. The sheath 8 is coaxial with the inner catheter 2. The sheath 8 is arranged to sheath the implant 20 until, in use, the sheath 8 is withdrawn proximally, relative to the implant 20 and the inner catheter 2, to release (deploy) the implant 20 at the site. The site is at a distal location. The implant 20 is typically located on the inner catheter at a distal location. Fig. 1 shows a partially withdrawn sheath 8. In Fig. 1, the sheath 8 has been withdrawn to beyond the distal end of the implant 20. The direction 12 which is in a direction along a longitudinal axis of at least one of the inner catheter 2 and the sheath 8 indicates the direction in which the sheath 8 is withdrawn.

It is seen that the sheath 8 is withdrawn towards a proximal location, i.e. towards a device handle.

In the embodiment, the cylindrical distal end component 4 includes a first plurality of implant retention elements 14. The first plurality of implant retention elements 14 during withdrawal of the sheath 8 protrude radially outward from the cylindrical distal end component 4 to engage a radially inward facing surface of the implant 20 to restrain the implant 20 from being carried by the sheath 8 proximally, relatively to the cylindrical distal end component 4. In the embodiment, the first plurality of implant retention elements 14 are formed from a portion of the wall 6 of the cylindrical distal end component 4. Further, the first plurality of implant retention elements are configured to utilise a shape memory effect to assume a radially outwardly protruding configuration to engage the radially inward facing surface of the implant prior to the withdrawal of the sheath.

In particular, in order to maintain defined radial stress conditions in the space accommodating the implant 20 between the inside of the sheath 8 and the outside of the inner catheter 2, it is beneficial to have at least one element, preferably a plurality of implant retention elements 14, within that space that are able to assume varying degrees of radial deflection without a significant change in the radial force to achieve these states of varying degrees of radial deflection. The shape memory effect of the plurality of implant retention elements 14 achieve this result. Each of the plurality of implant retention elements 14 have a plateau area in its load-deformation (also referred to as stress-strain) curve. The implant retention elements are constructed from a wall 6 of the inner catheter 2. In one embodiment, the inner catheter 2 is a nitinol alloy. The implant retention element(s) 14 are configured to utilize at least one of a superelastic and a shape memory property of the nitinol alloy. Preferably, the cylindrical distal end component 4 is made of nitinol.

In one embodiment, at least one of the first plurality of implant retention elements 14 is a flap. The flap comprises a hinge 16. The flap is arranged to extend distally from a root line in the cylindrical distal end component 4. In this way, the implant 20 is prevented from moving in a proximal direction.

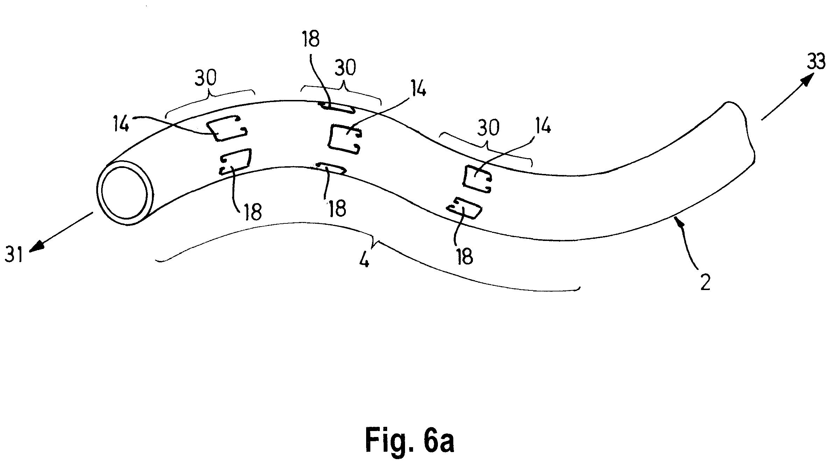

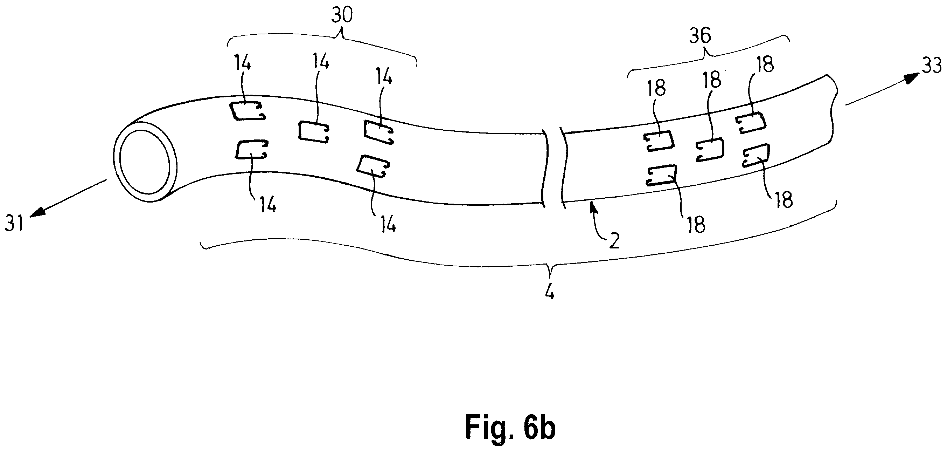

The cylindrical distal end component 4 may include a second plurality of implant retention elements 18 (see Figs. 6a and 6b). The second plurality of implant retention elements 18 are configured to utilise a shape memory effect to protrude radially outward from the cylindrical distal end component to engage a radially inward facing surface of the implant 20 to restrain the implant from being carried distally, relative to the cylindrical distal end component 4. In this way, the implant 20 is prevented from moving in a distal direction 31. The second plurality of implant retention elements 18 differ from the first plurality of implant retention elements 14 in as far as they are oriented in an opposite direction to the first plurality of implant retention elements 14. In particular, the flap is arranged to extend proximally from a root line in the cylindrical distal end component 4. In all other respects, in embodiments of the invention, they are the same as the first plurality of implant retention elements 14.

In embodiments comprising both a first and second plurality of implant retention elements 14, 18 for example, both distal and proximal flaps, the implant can be prevented from moving in both directions 31, 33.

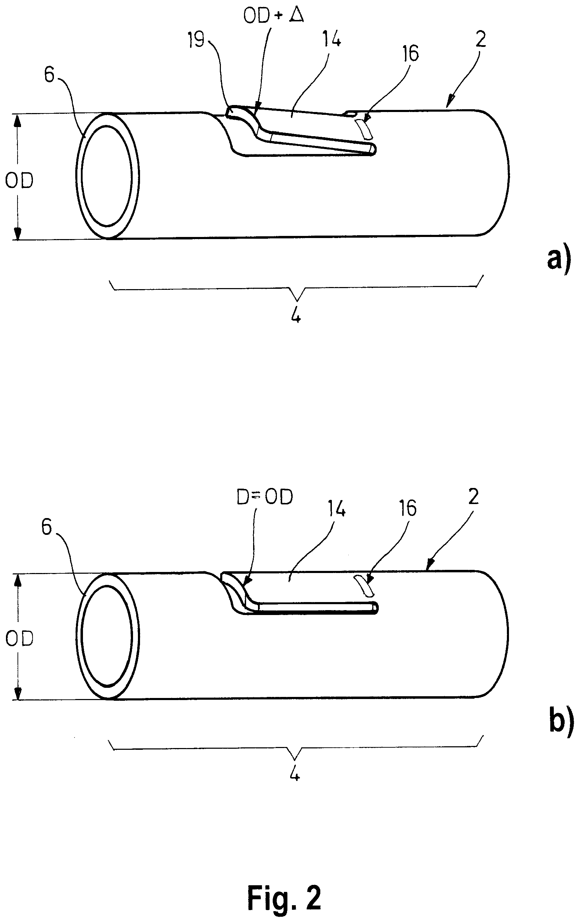

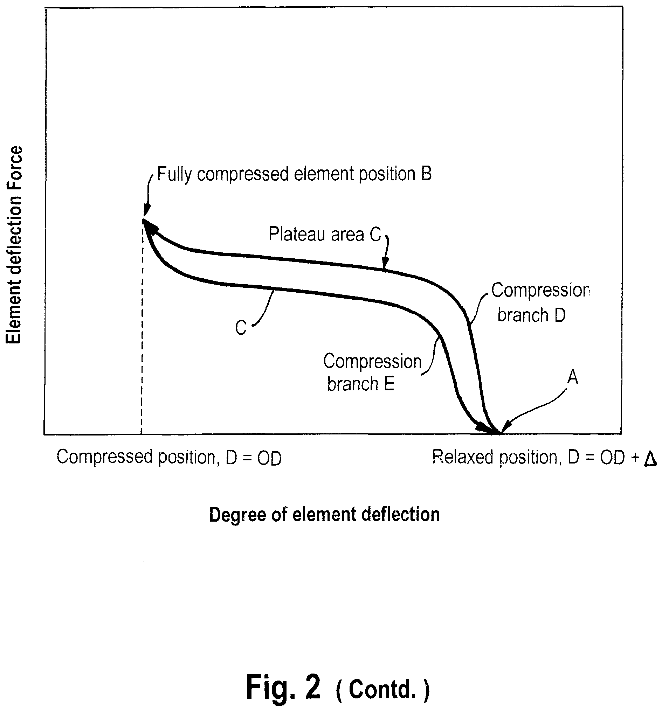

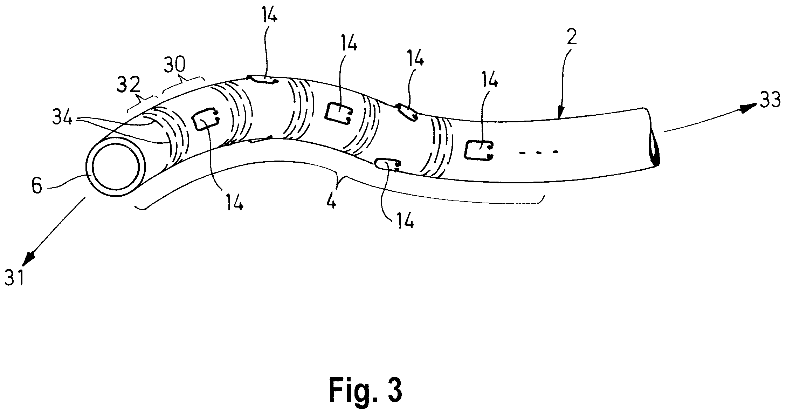

The inner catheter 2 shown in Figs. 2 and 3 show implant retention elements 14 pointing towards a distal end 31 of the delivery system, in other words, the radially outwardly protruding portion (for example, the deflected portion of the flap) is located distally with respect to the hinge 16. In this way, the implant retention features 14 are arranged to prevent the implant, for example, a stent or stent graft, from moving towards a proximal end 33 when the sheath 8 is retracted, as described above. In other embodiments (see Fig. 6), implant retention features 18 are provided that are arranged to point in the proximal direction 33, in other words, the radially outwardly protruding portion (for example, the deflected portion of the flap) is located proximally with respect to the hinge 16, to prevent the implant from moving towards the distal end 31 when the sheath 8 is retracted.

In one embodiment as shown in Fig. 6a, implant retention elements 14, 18 arranged to point in both directions 31, 33, respectively, may be provided in the same implant retention section 30, to prevent the implant 20 from moving in either direction 31, 33. Such an arrangement is advantageous, if after implant loading on the inner catheter 2, the ability to move the implant in either direction 31, 33 to precisely adjust its position in the sheath 8 is desired.

With further reference to Fig. 6a, for short implants, the implant retention elements 14 pointing towards the distal end 31 (a first plurality of retention elements 14) and the proximal end 33(a second plurality of retention elements 18), respectively, may be arranged in the same implant retention section 30 on the inner catheter 2. In one embodiment, the implant retention section 30 comprises a plurality of rows. The implant retention elements 14, 18 may be arranged in the rows 30 with alternative direction. In one embodiment, the first and second plurality of retention elements 14, 18 are arranged circumferentially around the cylindrical distal end component 4 in at least one row 30, wherein the first and second plurality of retention elements 14, 18 alternate along at least one row. Such an arrangement allows for adjustment of the location of the implant 20 relative to the sheath 8 (not relative to the inner catheter 2) in both proximal and distal directions.

With reference to Fig. 6b, for long implants, in one embodiment, in a first implant retention section 30 at a distal location corresponding to a distal end of the implant 20, implant retention elements 14 pointing in a distal direction 31 are provided and in a second implant retention section 36 at a relatively proximal location corresponding to a proximal end of the implant 20, implant retention elements 18 point in a proximal direction 33 are provided. In one embodiment, the first plurality of retention elements 14 is disposed at a location on the cylindrical distal end component 4 that is arranged to be received by a distal region of the implant 20. The second plurality of retention elements 18 are disposed at a location on the cylindrical distal end component 4 that is arranged to be received by a proximal region of the implant 20. In this way, whichever way the implant 20 is moved by pushing or pulling, the inner catheter 2 during implant position adjustment, it is ensured that the implant is pulled, rather than pushed, thus preventing buckling and seizing within the sheath 8.

With reference to Fig. 2, the shape memory property and the superelastic property of the plurality of implant retention elements are described. Fig. 2a shows an alloy tube which forms a portion of an inner catheter 2. In Fig. 2a, the implant retention element 14 is in a relaxed position. Fig. 2b shows the same alloy tube as Fig. 2a. In Fig. 2b, the implant retention element 14 is in a compressed position.

Figs. 2a and 2b show the shape of an implant retention element. By way of example, in Figs. 2a and 2b, a first implant retention element 14 is shown. However, the shape of the implant retention element shown in Figs. 2a and 2b applies to both the first and second implant retention elements 14, 18 and pluralities thereof, respectively. Similarly, the description hereinbelow relating to Figs. 2a, 2b and 2c applies to both the first and second (plurality of) implant retention elements 14, 18, respectively.

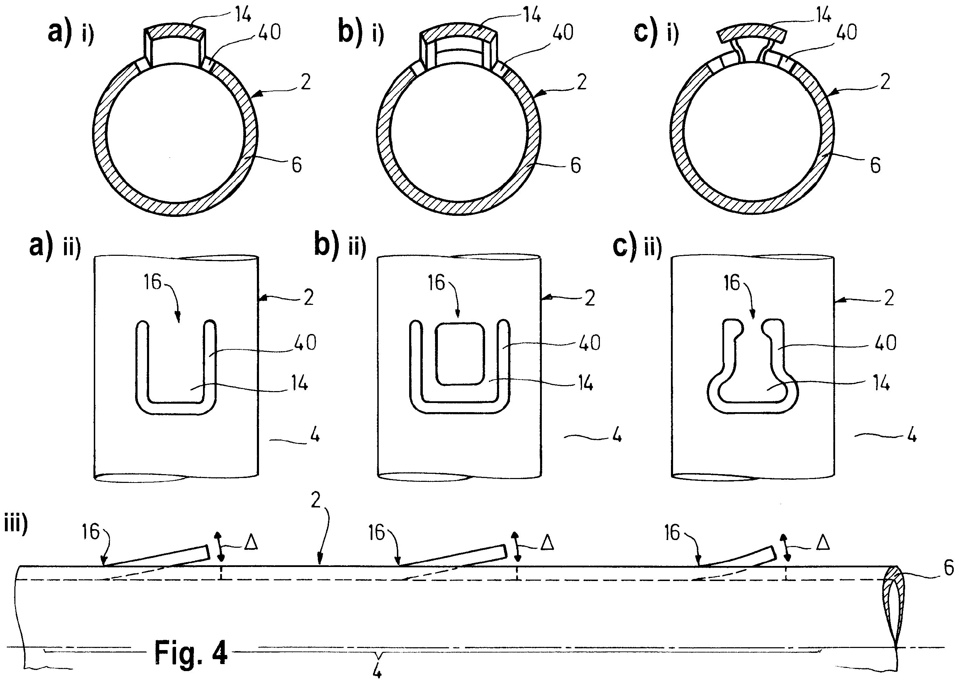

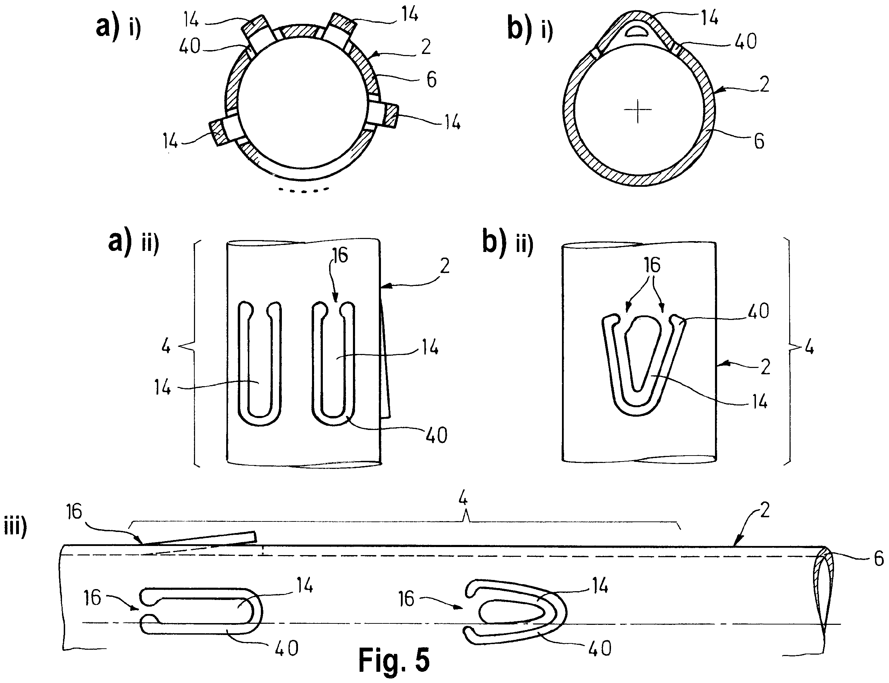

Fig. 2c is a graph showing a stress-strain curve of the alloy tube, in particular, the implant retention element 14 shown in Figs. 2a and 2b. Figs. 2a to 2c illustrate the stress of the implant retention feature 14 at a hinge 16. The hinge 16 is the section about which the main portion of deflection of the implant retention element 14 occurs, for example, the location of the deflection of the flap. The implant retention element 14 in the embodiment of Fig. 2a is a flap. The invention is not limited in this respect, as shown and described with reference to Figs. 4 and 5.

In the embodiment of Fig. 2, a flap is cut from an inner catheter wall 6, made of, for example, nitinol tubing. The nitinol tubing wall acts as the implant supporting inner catheter 2. The end of the flap shape is set in a position that extends slightly, by the distance delta beyond the original diameter OD of the nitinol tube from which the flap is cut. As a consequence, in Fig. 2a, the flap 14 is stress free at its hinge 16. As seen in Fig. 2a, the width of the tube including the extended flap is OD+delta. Fig. 2c illustrates how the implant retention element 14 exerts a relatively constant outward force over a large range of deflection after a little initial compression, once the stress state of the hinge reaches the plateau area C shown on the graph. As seen in Fig. 2c, the point A illustrates the stress-strain relationship of the implant retention element 14, 18 in Fig. 2a and the point B illustrates the stress-strain relationship of the implant retention element 14, 18 in Fig. 2b. In the plateau area C, regardless of the deflection of the implant retention element 14, 18 the stress exerted by the implant retention element 14, 18 is constant.

A method of making a shape memory alloy tube, for example a nitinol tube, for a catheter device is now described. In one embodiment, the method comprises forming a hinge 16 in the shape memory alloy tube 2. The method further comprises deflecting a portion of the shape memory alloy tube around the hinge 16, and setting the deformed portion by heating the shape memory alloy. In the embodiment shown in Fig. 2a, the deflection delta is the position in which the shape memory alloy tube is heat set. In this position, the shape memory alloy, at body temperature, can assume a configuration in which the portion is radially outwardly deflected from the shape memory alloy tube around the hinge 16. In the embodiment shown in Fig. 2a, the portion is deflected around the hinge 16 so that it outwardly deflects a distance delta.

The portion of the alloy tube deflected around the hinge is the implant retention element 14.

The shape memory alloy tube forms the inner catheter 2. The heat set portion of the alloy tube deflected around the hinge 16 forms the implant retention element 14.

The graph in Fig. 2c shows the implant retention element deflection force (y axis) with respect to the degree of implant retention element deflection (x axis).

Since the shape memory alloy tube is heat set in the deflected position, once the alloy tube is heat set, there is zero stress at the hinge. The hinge portion of the heat set tube shown in Fig. 2a is in its relaxed position. On the stress-strain curve shown in Fig. 2c, point A (identified by the arrow) depicts the relaxed position of the hinge portion of the heat set alloy tube depicted in Fig. 2a, point B (identified by the arrow) depicts the fully compressed position of the hinge portion of the heat set alloy tube depicted in Fig. 2b. In the fully compressed position there is maximum stress at the hinge. In the compressed position the diameter of the alloy tube is equal to the original diameter OD and the deflection (delta) is zero degrees. It can be seen from the graph in Fig. 2c that there are plateau areas C (identified by the arrow) in which the deflection force exerted by the implant retention element remains substantially constant regardless of the degree of deflection of the implant retention element 14. In this way, regardless of the extent to which the implant retention element is deflected, i.e. regardless of the extent to which the implant retention element 14 extends into the implant 20 or a covering of the implant, the force exerted on the implant 20 or the covering of the implant remains substantially constant, so that the implant 20 can be more reliably retained when the sheath 8 is withdrawn.

As shown in Fig. 2c, the alloy tube, for example, the nitinol tube, exhibits hysteresis. It is seen that there is a compression branch D and an expansion branch E. In both branches there is a plateau area C. As described above, in embodiments, the implant retention elements 14, 18, utilize this plateau area C. The stress-strain state of the material follows difference curve branches depending on the direction of the deformation, A to B or B to A.

According to an embodiment of the present invention, there is a method of assembling a catheter assembly for transluminally delivering a self-expanding tubular implant to a site in a body. The catheter assembly comprises an inner catheter 2 comprising a shape memory alloy tube 2, a sheath 8 and a self-expanding implant 20. The method comprises the steps of: assembling the self-expanding implant 20 on the shape memory alloy tube 2, wherein in the assembling step, the shape memory alloy tube 2 is at a temperature which allows it to accommodate predominantly by martensite twinning, the radially inward pressure imposed on it by the self-expanding implant 20. In this way, the shape memory alloy tube 2 is able to retain the self-expanding implant 20 on the inner catheter 2.

The overall number of implant retention elements, their size, their density, i.e. their circumferential and longitudinal spacing, the shape and roundness of their leading edge and their bending stiffness, which is determined by the design of the hinge 16, influence their ability to reliably anchor the implant on the inner catheter 2 while not damaging the delicate implant covering during loading, implant positioning, device handling, tracking and deployment. Depending on the particular application and implant to be deployed, the shape, number and arrangement etc. of the implant retention elements can be chosen accordingly. One arrangement is shown, by way of example, in Fig. 3. Figs. 4 and 5 depict some further examples of implant retention features according to embodiments of the present invention and are described in further detail hereinbelow.

In a further embodiment, pluralities of at least the first and second plurality of implant retention elements 14, 18 are arranged along a length of the cylindrical distal end component 4, so that a force exerted on the implant 20 by the at least first and second plurality of implant retention elements 14, 18 is distributed evenly along a length of an implant 20.

The first and second plurality of implant retention elements 14, 18 are not necessarily arranged at the ends of the compressed implant 20, instead, there may be several pluralities arranged along the length of a long implant 20 to deal with the fact that a plurality of implant retention elements 14, 18 arranged under a distal end of a long implant 20 disengages from the implant 20 once its distal end is deployed, a further plurality of implant retention elements may therefore, be arranged further proximally, but not all the way at the most proximal end of the implant 20, is then arranged to take over the retention of the implant 20, whilst the sheath 8 is being withdrawn across the implant 20.

With further reference, for example, to Fig. 2, the flap 14 may comprise a hinge portion 16 about which the flap 14 is arranged to deflect, and an engaging portion 19 arranged to engage the implant. The hinge portion 16 may extend along a root line and the engaging portion 19 may extend along a contact line for contacting the implant 20. In one embodiment, the root line of the hinge portion 16 is shorter than the contact line of the engaging portion 19 in a circumferential direction. In this way, the force exerted on the implant 20, for example, a covered implant covered in a delicate ePTFE coating, is distributed. In this way, damage to the covering can be avoided. In one embodiment, the contact line may be up to twice as long as the root line. In this way, damage to a delicate ePTFE coating is yet further reduced.

Fig. 3 shows an inner catheter according to an embodiment of the invention. In Fig. 3 the cylindrical distal end component 4 is arranged to receive an implant 20. The implant 20 is disposed on the cylindrical distal end component 4. The cylindrical distal end component 4 may also be referred to as the implant section of the inner catheter 2, since it is the section of the inner catheter 2 that receives the implant. In one embodiment the cylindrical distal end component 4 (implant section) is constructed from a nitinol tube, as shown in Fig. 3. The tube is divided into sections 30 that bear the implant retention features 14. These sections 30 are linked by flexible sections 32 of nitinol tube.

In other words, the cylindrical distal end component 4 comprises at least one implant retention section 30 having at least one of the first and/or second plurality of implant retention elements 14, 18 and at least one flexible section 32. Preferably, the at least one flexible section 32 comprises a plurality of staggered circumferentially extending slits 34 to form a cardanic joint. The slits 34 endow the inner catheter 2 with enhanced bending ability, thereby facilitating advance of the distal end of the catheter device along especially tortuous lumens to hard to reach implant sites in the body. The slits 34 may be laser cuts. In one embodiment, see Fig. 3, at least one implant retention section 30 and at least one flexible section 32 alternate along the length of the cylindrical distal end component 4.

Fig. 4 shows various embodiments and various views of the various embodiments showing a variety of retention features according to the various embodiments of the invention. Fig. 5 shows various further embodiments and various views of the various embodiments showing a variety of retention features according to the various embodiments of the invention.

As seen in Figs. 4 and 5, the implant retention elements 14 can have various shapes. For example, the implant retention elements 14 may be flaps (see Fig. 4a and Fig. 4b), arches (see Fig. 4b), chevrons (see Fig. 5b), barbs or spikes (Fig. 5a) . The various shapes may be used on their own or in combination with other shapes.

In more detail, with reference to Figs. 4 and 5: Figs. 4a to c and Figs. 5 a and b show various shapes of the implant retention features. Figs. 4i) and Figs. 5i) show a cross section of the alloy tube for each shape, respectively. Figs.4ii) and 5ii) show a plan view of the alloy tube for each shape, respectively. Figs. 4iii) and Figs. 5iii) show a side view of the alloy tube for each of the shapes, respectively.

The various shapes may be formed by laser cutting. The laser cuts 40 for each of the various shapes, respectively, are shown in Figs. 4 and 5. Once the appropriate laser cuts 40 have been made, depending on the desired shape, the implant retention features 14 are shape set such that the portion of, for example the flap, remote from the hinge 16, where the implant retention features are linked to the alloy tube 2, slightly protrude beyond the original diameter OD by a distance delta. The angle at which the implant retention elements 14 protrude from the body of the alloy tube 2 is adjusted such that when the elements are compressed by the implant 20 crimped onto it, the area 16 where the implant retention features 14 are joined, i.e. hinged at the hinge 16, to the body of the alloy tube are in the region of the plateau area C (see Fig. 2c) of the material's stress-strain curve (see Fig. 2c). For example, in the embodiment where the alloy tube is a nitinol tube. The implant retention features 14 are shape set so that the hinge 16 is in the region of the plateau area of nitinol's stress-strain curve. In this way, the first and/or second plurality of retention elements 14, 18 are configured so that a force exerted on the implant by the first and/or second retention elements, respectively, within a range of deflection of the hinge is substantially the same over the range of deflection. An advantage of this arrangement is that regardless of the deflection, the force exerted on the implant is the same, therefore tolerances are not so critical.

In an embodiment, the hinge portion 16 is substantially in a plateau area C (region) of the stress-strain curve of the material of the at least first and second plurality of implant retention elements 14, when engaged with the implant 20, so that the at least first and second plurality of implant retention elements 14 exert more or less the same force on the implant 20 regardless of how much they protrude (deflect) from the cylindrical distal end component 4.

With regard to the various shapes of the implant retention elements 14, 18 envisaged, in one embodiment, the flap 14 extends in an arc around the circumference of the cylindrical distal end component 4. As described above, the first and/or second retention elements 14, 18 may assume a configuration in which the first and/or second retention elements 14 are deflected about the hinge 16. The hinge 16 may be referred to as a hinge portion 16.

In one embodiment, the first and/or second plurality of retention elements 14, 18 are unpolished. In this way, an implant, in particular and for example, an uncovered stent, for example an uncovered nitinol stent, can be retained more effectively, since the relatively rough surface of the implant retention element 14 can more readily engage with the structure of the uncovered stent.

In embodiments, the first and/or second plurality of implant retention elements 14, 18 are able to lie within the wall thickness. In this way, the implant retention elements 14, 18 require no space of their own, therefore, the diameter of the catheter device is reduced.

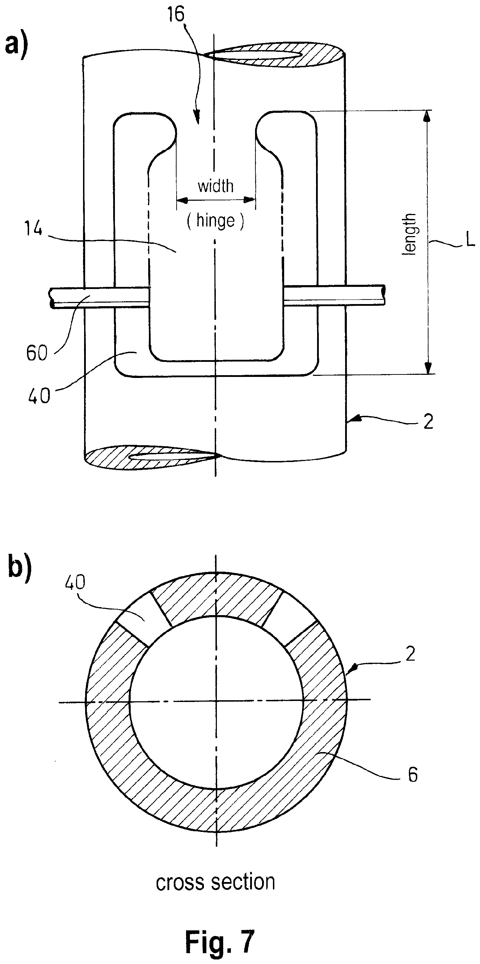

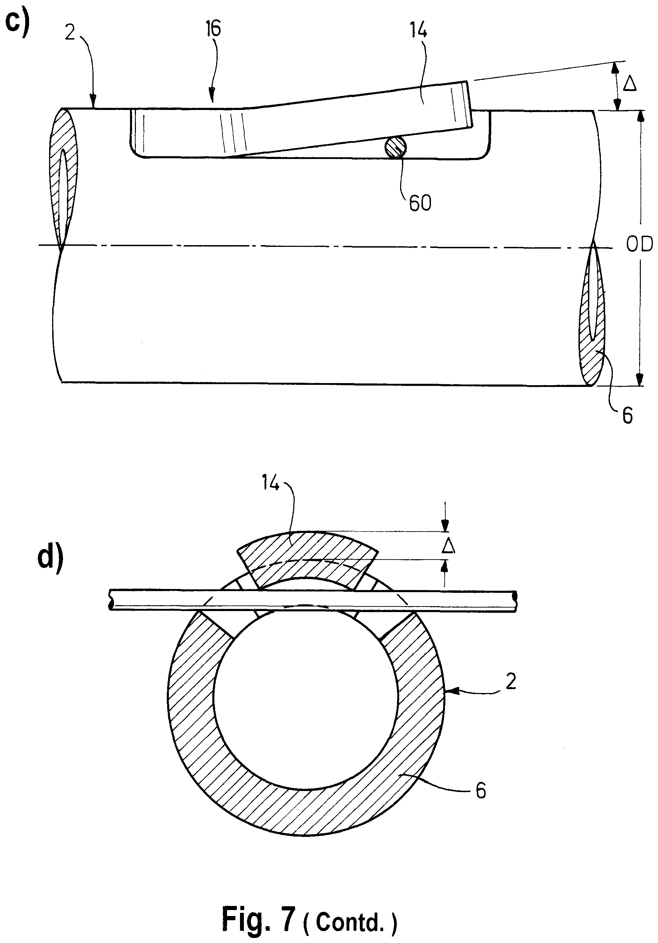

Fig. 7 shows a retention feature according to an embodiment of the present invention and a method of making a shape memory alloy tube according to an embodiment of the present invention. In particular, Fig. 7a shows a plan view of an alloy tube in which an implant retention feature 14 is to be formed. In the embodiment shown in Fig. 7, the implant retention feature 14 is in the form of a flap having a length L. The length L extends from the base of the hinge 16 to the extent of the laser cut 40 in the longitudinal direction of the alloy tube 2. In Fig. 7a, a length of wire 60 is shown. The wire 60 is used in the formation of the implant retention element 14, and is described in more detail below.

Fig. 7b shows a cross section of the alloy tube shown in Fig. 7a without the wire 60. In Fig. 7b, the laser cut 40 is seen in cross section. The flap has not yet been deflected or shape set.

Fig. 7c shows a side view of the alloy tube 2 shown in Fig. 7a and Fig. 7d shows a cross section of the alloy tube 2 shown in Fig. 7a.

With reference to Figs. 7a to 7d, it is seen that the flap can be positioned for shape setting with its free end (the end remote from the hinge 16) protruding a distance delta beyond the original diameter OD of the alloy tube 2. This may be achieved, as shown in Figs. 7a, 7c and 7d by inserting a length of wire 60 across. In the embodiment shown in Fig. 7, a section of nitinol material has been cut away by the laser cut 40 on either side of the flap, rather than just a single pass laser cut such as across the end of the flap. In this way easy insertion of a lifting tool prior to inserting the wire 60 is achieved. The implant retention element 14, 18 is constructed such that the hinge portion 16 of the implant retention element 14, 18 is in a stress state corresponding to the plateau area C (see Fig. 2c) of the deformation curve when an implant is crimped around it. The width of the hinge 16 determines the level of force that the flap 14 is able to exert when being compressed inward, that is the bending stiffness of the implant retention element 14, 18.

Further disclosure includes a catheter device for transluminally delivering a self-expanding tubular implant to a site in a body. The device comprises: an inner catheter comprising a cylindrical distal end component with a wall thickness, wherein the cylindrical distal end component is arranged, in use, to be received within the lumen of the implant. The catheter device further comprises: a sheath, coaxial with the inner catheter, wherein the sheath is arranged to sheath the implant until, in use, the sheath is withdrawn proximally, relative to the implant and the inner catheter, to release (deploy) the implant at the site, wherein the cylindrical distal end component includes a first plurality of implant retention elements, which, during withdrawal of the sheath protrude radially outwardly from the cylindrical distal end component to engage a radially inward facing surface of the implant to restrain the implant from being carried by the sheath proximally, relatively to the cylindrical distal end component. The first plurality of implant retention elements are able to lie within the wall thickness, and in that: the first plurality of implant retention elements have a shape memory and utilise a shape memory effect to assume, at body temperature, a radially outwardly protruding configuration effective to engage the radially inward facing surface of the implant prior to the withdrawal of the sheath. In this way, it is possible to achieve a more compact device.

In a further embodiment, the first plurality of implant retention elements lie within the wall thickness at a temperature at which the implant is stored on the inner catheter.

Having an austenite finish temperature (Af temperature) below body temperature may serve to reduce the force exerted onto the inner wall of a (covered implant) during storage of the device at room temperature. In such a case, loading of the implant is performed a temperature above Af, if it is desired to use the "grip" of the elements to transfer the implant from the closed crimp head to the sheath. During storage at a temperature below Af the radial force exerted is reduced which is beneficial as polymers show a time dependent deformation behaviour (creep) and as such, persistent pressure exerted by the retention elements onto the inside covering of the implant may lead to local damage of the covering.

When deploying the device at body temperature, above Af, the full radial force exerted by the retention elements is available.

The catheter device hereindescribed can be used by a medical practitioner to deploy an implant 20 at an implant site in a body. Before use, the catheter device is stored in packaging (not shown). The practitioner removes the catheter device from the packaging. The practitioner advances the catheter device along a bodily lumen to the implant site. Once the implant site has been reached, the other sheath 8 is withdrawn in a proximal direction by the practitioner. By withdrawing the sheath 8, the implant 20 is deployed at the implant site.

The invention is not limited to the embodiments shown and described above.

## Claims

1. A catheter device for transluminally delivering a self-expanding tubular implant to a site in a body, the device comprising:
an inner catheter (2) comprising a cylindrical distal end component (4) with a wall (6), wherein the cylindrical distal end component (4) is arranged, in use, to be received within the lumen of the implant (20); and
a sheath (8), coaxial with the inner catheter (2), wherein the sheath (8) is arranged to sheath the implant (20) until, in use, the sheath (8) is withdrawn proximally, relative to the implant (20) and the inner catheter (2), to release (deploy) the implant (20) at the site,
wherein the cylindrical distal end component (4) includes a first plurality of implant retention elements (14), which, during withdrawal of the sheath (8) protrude radially outwardly from the cylindrical distal end component (4) to engage a radially inward facing surface of the implant (20) to restrain the implant (20) from being carried by the sheath (8) proximally, relative to the cylindrical distal end component (4), wherein the first plurality of implant retention elements (14) are formed from a portion of the wall (6) of the cylindrical distal end component (4), wherein the first plurality of implant retention elements (14) are configured to utilise a shape memory effect to assume a radially outwardly protruding configuration to engage the radially inward facing surface of the implant (20) prior to the withdrawal of the sheath (8), wherein at least one of the first plurality of implant retention elements (14) is a flap, wherein the flap comprises a hinge portion (16) about which the flap is arranged to deflect, and an engaging portion (19) arranged to engage the implant (20), and wherein the hinge portion (16) extends along a root line and the engaging portion (19) extends along a contact line for contacting the implant (20), **characterized in that**: the flap is arranged to extend distally from the root line in the cylindrical distal end component (4), and **in that**: the root line of the hinge portion (16) is shorter than the contact line of the engaging portion (19) in a circumferential direction.

2. The catheter device according to claim 1, wherein the cylindrical distal end component (4) includes a second plurality of implant retention elements (14), which are configured to utilise a shape memory effect to protrude radially outward from the cylindrical distal end component (4) to engage a radially inward facing surface of the implant (20) to restrain the implant (20) from being carried distally, relative to the cylindrical distal end component (4) .

3. The catheter device according to claim 2, wherein at least one of the second plurality of implant retention elements (14) is a flap arranged to extend proximally from a root line in the cylindrical distal end component (4).

4. The catheter device according to any preceding claim, wherein pluralities of at least the first and second plurality of implant retention elements (14) are arranged along a length of the cylindrical distal end component (4), so that a force exerted on the implant (20) by the at least first and second plurality of implant retention elements (14) is distributed evenly along a length of an implant (20).

5. The catheter device according to claim 1, wherein the contact line is up to twice as long as the root line.

6. The catheter device according to any preceding claim, wherein the cylindrical distal end component (4) is made of nitinol.

7. The catheter device according to any preceding claim, wherein the cylindrical distal end component (4) comprises at least one implant retention section (30, 36) having at least one of the first and/or second plurality of implant retention elements (14) and at least one flexible section (32).

8. The catheter device according to a claim 7, wherein at least one flexible section (32) comprises a plurality of staggered circumferentially extending slits (34) to form a cardanic joint.

9. The catheter device according to any of the preceding claims 4 to 7, wherein at least one implant retention section (30, 36) and at least one flexible section (32) alternate along the length of the cylindrical distal end component (4).

10. The catheter device according to any of the preceding claims, wherein the cylindrical distal end component (4), wherein at least the first plurality of retention elements (14) and the second plurality of retention elements (14) are disposed in the same implant retention section (30, 36).

11. The catheter device according to claim 10, wherein the first and second plurality of retention elements (14) are arranged circumferentially around the cylindrical distal end component (4) in at least one row, wherein the first and second plurality of retention elements (14) alternate along at least one row.

12. The catheter device according to any of the preceding claims, wherein the first plurality of retention elements (14) is disposed at a location on the cylindrical distal end component (4) that is arranged to be received by a proximal region of the implant (20).

13. The catheter device according to any of the preceding claims, wherein the second plurality of retention elements (14) are disposed at a location on the cylindrical distal end component that is arranged to be received by a distal region of the implant (20).

14. The catheter device according to claim 12, wherein the flap extends in an arc around the circumference of the cylindrical distal end component (4).

15. The catheter device according to any of the preceding claims, wherein the first and/or second plurality of retention elements (14) assume a configuration in which the first and/or second retention elements are deflected about the hinge portion (16).

16. The catheter device according to any preceding claim, wherein the first and/or second plurality of retention elements (14) are configured so that a force exerted on the implant (20) by the first and/or second plurality of retention elements (14), respectively, within a range of deflection of the hinge portion (16) is substantially the same over the range of deflection.

17. The catheter according to any preceding claims, wherein the flap is configured to be at least one of a barb, an arch, a chevron or a spike.

18. The catheter according to any preceding claim, wherein the first and/or second plurality of retention elements (14) are unpolished.

19. The catheter device according to any preceding claim, wherein the first plurality of implant retention elements (14) are able to lie within the wall thickness.

20. The catheter device according to any of the preceding claims, wherein the hinge portion (16) is substantially in a plateau region of the stress-strain curve of the material of the at least first and second plurality of implant retention elements (14), when engaged with the implant (20), so that the at least first and second plurality of implant retention elements (14) exert more or less the same force on the implant (20) regardless of how much they protrude (deflect) from the cylindrical distal end component (4).

21. The catheter device according to any of the preceding claims, wherein the root line of the hinge portion (16) is shorter than the contact line of the engaging portion (19) in a circumferential direction, so that the force exerted on a covered implant covered in an ePTFE coating is distributed.

22. A method of making a shape memory alloy tube for a catheter device for transluminally delivering a self-expanding tubular implant to a site in a body, the method comprising: forming a hinge portion (16) in the shape memory alloy tube, deforming the shape memory alloy tube by deflecting a portion of the shape memory alloy tube around the hinge, and setting the deformed portion by heating the shape memory alloy, so that the shape memory alloy, at body temperature, can assume a configuration in which the hinge portion (16) is radially outwardly deflected from the shape memory alloy tube, wherein the hinge portion (16) is comprised in a flap (14), about which hinge portion (16) the flap (14) is arranged to deflect, and an engaging portion (19) arranged to engage the implant, wherein the hinge portion (16) extends distally along a root line and the engaging portion (19) extends along a contact line for contacting the implant (20), and wherein the root line of the hinge portion (16) is shorter than the contact line of the engaging portion (19) in a circumferential direction.

23. A method of assembling a catheter assembly for transluminally delivering a self-expanding tubular implant to a site in a body, the catheter assembly comprising: an inner catheter (2) comprising a shape memory alloy tube, a sheath (8) and a self-expanding implant (20), the method comprising the steps of: assembling the self-expanding implant on the shape memory alloy tube, wherein in the assembling step, the shape memory alloy tube is at a temperature which allows it to accommodate predominantly by martensite twinning, the radially inward pressure imposed on it by the self-expanding implant, wherein a flap (14) is provided comprising a hinge portion (16) about which the flap is arranged to deflect, and an engaging portion (19) arranged to engage the implant (20), wherein the hinge portion (16) extends along a root line and the engaging portion (19) extends along a contact line for contacting the implant (20), and wherein the root line of the hinge portion (16) is shorter than the contact line of the engaging portion (19) in a circumferential direction.

## Patentansprüche

1. Kathetervorrichtung zur transluminalen Abgabe eines selbstexpandierenden rohrförmigen Implantats an eine Stelle in einem Körper, wobei die Vorrichtung umfasst:
einen inneren Katheter (2), der eine zylindrische distale Endkomponente (4) mit einer Wand (6) umfasst, wobei die zylindrische distale Endkomponente (4) im Gebrauch angeordnet ist, um innerhalb des Lumens des Implantats (20) aufgenommen zu werden; und
eine Hülle (8), koaxial zum inneren Katheter (2), wobei die Hülle (8) angeordnet ist, um das Implantat (20) zu umhüllen, bis die Hülle (8) im Gebrauch proximal in Bezug auf das Implantat (20) und den inneren Katheter (2) abgezogen wird, um das Implantat (20) an der Stelle freizugeben (einzusetzen),
wobei die zylindrische distale Endkomponente (4) eine erste Vielzahl von Implantat-Rückhalteelementen (14) umfasst, die beim Abziehen der Hülle (8) radial nach außerhalb aus der zylindrischen distalen Endkomponente (4) hervorstehen, um in eine radial nach innen zeigende Oberfläche des Implantats (20) einzugreifen, um das Implantat (20) davon abzuhalten, von der Hülle (8) proximal, in Bezug auf die zylindrische distale Endkomponente (4), mitgeführt zu werden, wobei die erste Vielzahl von Implantat-Rückhalteelementen (14) aus einem Abschnitt der Wand (6) der zylindrischen distalen Endkomponente (4) gebildet wird, wobei die erste Vielzahl von Implantat-Rückhalteelementen (14) konfiguriert ist, um einen Formgedächtniseffekt zu nutzen, um eine radial nach außen vorstehende Konfiguration anzunehmen, um vor dem Abziehen der Hülle (8) in die radial nach innen zeigende Oberfläche des Implantats (20) einzugreifen, wobei mindestens eines von der Vielzahl von Implantat-Rückhalteelementen (14) eine Klappe ist, wobei der Klappe einen Gelenkabschnitt (16) umfasst, um den herum die Klappe zum Ablenken angeordnet ist, und einen Eingriffsabschnitt (19), der angeordnet ist, um in das Implantat (20) einzugreifen, und wobei sich der Gelenkabschnitt (16) entlang einer Fußlinie erstreckt, und sich der Eingriffsabschnitt (19) entlang einer Berührungsinie erstreckt, um das Implantat (20) zu berühren, **dadurch gekennzeichnet, dass**: die Klappe angeordnet ist, um sich distal aus der Fußlinie in der zylindrischen distalen Endkomponente (4) zu erstrecken, und dadurch, dass: die Fußlinie des Gelenkabschnitts (16) kürzer als die Berührungsinie des Eingriffsabschnitts (19) in einer Umfangsrichtung ist.

2. Kathetervorrichtung nach Anspruch 1, wobei die zylindrische distale Endkomponente (4) eine zweite Vielzahl von Implantat-Rückhalteelementen (14) umfasst, die konfiguriert sind, um einen Formgedächtniseffekt zu nutzen, um radial nach außen aus der zylindrischen distalen Endkomponente (4) hervor zu stehen, in die radial nach innen zeigende Oberfläche des Implantats (20) einzugreifen, um das Implantat (20) davon abzuhalten, distal in Bezug auf die zylindrische distale Endkomponente (4) mitgeführt zu werden.

3. Kathetervorrichtung nach Anspruch 2, wobei mindestens eines der zweiten Vielzahl von Implantat-Rückhalteelementen (14) eine Klappe ist, die angeordnet ist, um sich proximal aus einer Fußlinie in der zylindrischen distalen Endkomponente (4) zu erstrecken.

4. Kathetervorrichtung nach einem vorstehenden Anspruch, wobei Vielzahlen von mindestens der ersten und zweiten Vielzahl von Implantat-Rückhalteelementen (14) entlang einer Länge der zylindrischen distalen Endkomponente (4) angeordnet sind, sodass sich eine Kraft, die durch die mindestens erste und zweite Vielzahl von Implantat-Rückhalteelementen (14) auf das Implantat (20) ausgeübt wird, gleichmäßig entlang einer Länge des Implantats (20) verteilt.

5. Kathetervorrichtung nach Anspruch 1, wobei die Berührungslinie bis zu doppelt so lang wie die Fußlinie ist.

6. Kathetervorrichtung nach einem vorstehenden Anspruch, wobei die zylindrische distale Endkomponente (4) aus Nitinol gefertigt ist.

7. Kathetervorrichtung nach einem vorstehenden Anspruch, wobei die zylindrische distale Endkomponente (4) mindestens einen Implantat-Rückhalteabschnitt (30, 36) umfasst, der mindestens eines von der ersten und/oder zweiten Vielzahl von Implantat-Rückhalteelementen (14) und mindestens einem flexiblen Abschnitt (32) aufweist.

8. Kathetervorrichtung nach eine Anspruch 7, wobei mindestens ein flexibler Abschnitt (32) eine Vielzahl von gestaffelten, sich im Umfang erstreckenden Schlitzen (34) umfasst, um ein kardanisches Gelenk zu bilden.

9. Kathetervorrichtung nach einem der vorstehenden Ansprüche 4 bis 7, wobei mindestens ein Implantat-Rückhalteabschnitt (30, 36) und mindestens ein flexibler Abschnitt (32) einander entlang der Länge der zylindrischen distalen Endkomponente (4) abwechseln.

10. Kathetervorrichtung nach einem der vorstehenden Ansprüche, wobei die zylindrische distale Endkomponente (4), wobei mindestens die erste Vielzahl von Rückhalteelementen (14) und die zweite Vielzahl von Rückhalteelementen (14) in demselben Implantat-Rückhalteabschnitt (30, 36) angeordnet sind.

11. Kathetervorrichtung nach Anspruch 10, wobei die erste und zweite Vielzahl von Rückhalteelementen (14) im Umfang um die zylindrische distale Endkomponente (4) herum, in mindestens einer Reihe angeordnet sind, wobei die erste und zweite Vielzahl von Rückhalteelementen (14) einander entlang mindestens einer Reihe abwechseln.

12. Kathetervorrichtung nach einem der vorstehenden Ansprüche, wobei die erste Vielzahl von Rückhalteelementen (14) an einer Stelle auf der zylindrischen distalen Endkomponente (4) angeordnet sind, die angeordnet ist, um durch einen proximalen Bereich des Implantats (20) aufgenommen zu werden.

13. Kathetervorrichtung nach einem der vorstehenden Ansprüche, wobei die zweite Vielzahl von Rückhalteelementen (14) an einer Stelle auf der zylindrischen distalen Endkomponente angeordnet sind, die angeordnet ist, um durch einen distalen Bereich des Implantats (20) aufgenommen zu werden.

14. Kathetervorrichtung nach Anspruch 12, wobei sich die Klappe in einem Bogen um den Umfang der zylindrischen distalen Endkomponente (4) herum erstreckt.

15. Kathetervorrichtung nach einem der vorstehenden Ansprüche, wobei die erste und/oder zweite Vielzahl von Rückhalteelementen (14) eine Konfiguration annehmen, in der das erste und/oder zweite Rückhalteelement um den Gelenkabschnitt (16) abgelenkt sind.

16. Kathetervorrichtung nach einem vorstehenden Anspruch, wobei die erste und/oder zweite Vielzahl von Rückhalteelementen (14) konfiguriert sind, sodass eine Kraft, die jeweils durch die erste und/oder zweite Vielzahl von Rückhalteelementen (14) auf das Implantat (20) ausgeübt wird, innerhalb eines Ablenkungsbereichs des Gelenkabschnitts (16) über den Ablenkbereich im Wesentlichen dieselbe ist.

17. Kathetervorrichtung nach einem der vorstehenden Ansprüche, wobei die Klappe konfiguriert ist, um mindestens eines von einem Widerhaken, einem Bogen, einem Winkel oder einem Dorn zu sein.

18. Kathetervorrichtung nach einem vorstehenden Anspruch, wobei die erste und/oder zweite Vielzahl von Rückhalteelementen (14) nicht poliert sind.

19. Kathetervorrichtung nach einem vorstehenden Anspruch, wobei die erste Vielzahl von Rückhalteelementen (14) imstande sind, innerhalb der Dicker der Wand zu liegen.

20. Kathetervorrichtung nach einem der vorstehenden Ansprüche, wobei der Gelenkabschnitt (16) im Wesentlichen in einem Plateau-Bereich der Spannungs-Dehnungs-Kennlinie des Materials der mindestens ersten und zweiten Vielzahl von Implantat-Rückhalteelementen (14) liegt, wenn er in das Implantat (20) eingreift, sodass die mindestens erste und zweite Vielzahl von Implantat-Rückhalteelementen (14) mehr oder weniger dieselbe Kraft auf das Implantat (20) ungeachtet davon ausüben, um wie viel sie aus der zylindrischen distalen Endkomponente (4) hervorstehen (ablenken).

21. Kathetervorrichtung nach einem der vorstehenden Ansprüche, wobei die Fußlinie des Gelenkabschnitts (16) kürzer als die Kontaktlinie des Eingriffsabschnitts (19) in einer Richtung des Umfangs ist, sodass die Kraft, die auf ein abgedecktes Implantat ausgeübt wird, das in einer ePTFE Beschichtung abgedeckt ist, verteilt wird.

22. Verfahren zur Herstellung eines Legierungsrohres mit Formgedächtnis für eine Kathetervorrichtung zur transluminalen Abgabe eines selbstexpandierenden rohrförmigen Implantats an eine Stelle in einem Körper, wobei das Verfahren umfasst: Bilden eines Gelenkabschnitts (16) in dem Legierungsrohr mit Formgedächtnis, Verformen des Legierungsrohres mit Formgedächtnis durch Ablenken eines Abschnitts des Legierungsrohres mit Formgedächtnis um das Gelenk herum, und Einstellen des verformten Abschnitts durch Erhitzen der Legierung mit Formgedächtnis, sodass die Legierung mit Formgedächtnis auf Körpertemperatur eine Konfiguration annehmen kann, in der der Gelenkabschnitt (16) radial nach außen aus dem Legierungsrohr mit Formgedächtnis abgelenkt wird, wobei der Gelenkabschnitt (16) in einer Klappe (14) enthalten ist, um welchen Gelenkabschnitt (16) herum die Klappe (14) zum Ablenken angeordnet ist, und einen Eingriffsabschnitt (19), der angeordnet ist, um in das Implantat einzugreifen, wobei sich der Gelenkabschnitt (16) distal entlang einer Fußlinie erstreckt, und sich der Eingriffsabschnitt (19) entlang einer Berührungslinie erstreckt, um das Implantat (20) zu berühren, und wobei die Fußlinie des Gelenkabschnitts (16) kürzer als die Berührungslinie des Eingriffsabschnitts (19) in einer Umfangsrichtung ist.

23. Verfahren zur Montage einer Kathetermontage zur transluminalen Abgabe eines selbstexpandierenden rohrförmigen Implantats an eine Stelle in einem Körper, wobei die Kathetermontage umfasst: einen inneren Katheter (2), der ein Legierungsrohr mit Formgedächtnis, eine Hülle (8) und ein selbstexpandierendes Implantat (20) umfasst, wobei das Verfahren die folgenden Schritte umfasst: Montieren des selbstexpandierenden Implantats auf das Legierungsrohr mit Formgedächtnis, wobei das Legierungsrohr mit Formgedächtnis in dem Montageschritt auf einer Temperatur ist, die es ihm erlaubt, um vorwiegend durch Martensit-Twinning den radial nach innen wirkenden Druck aufzunehmen, der ihm durch das selbstexpandierende Implantat auferlegt wird, wobei eine Klappe (14) vorgesehen ist, die einen Gelenkabschnitt (16) umfasst, um den herum die Klappe zum Ablenken angeordnet ist, und einen Eingriffsabschnitt (19), der angeordnet ist, um in das Implantat (20) einzugreifen,
wobei sich der Gelenkabschnitt (16) entlang einer Fußlinie erstreckt, und sich der Eingriffsabschnitt (19) entlang einer Berührungslinie erstreckt, um das Implantat (20) zu berühren, und wobei die Fußlinie des Gelenkabschnitts (16) kürzer als die Berührungslinie des Eingriffsabschnitts (19) in einer Umfangsrichtung ist.

## Revendications

1. Dispositif cathéter pour la pose par voie transluminale d'un implant tubulaire auto-déployable à un site dans un corps, le dispositif comprenant :
un cathéter interne (2) comprenant un composant d'extrémité distale cylindrique (4) avec une paroi (6), dans lequel le composant d'extrémité distale cylindrique (4) est agencé, en utilisation, pour être reçu à l'intérieur de la lumière de l'implant (20) ; et
une gaine (8), coaxiale par rapport au cathéter interne (2), dans lequel la gaine (8) est agencée pour envelopper l'implant (20) jusqu'à ce que, en utilisation, la gaine (8) soit retirée de manière proximale, par rapport à l'implant (20) et au cathéter interne (2), pour libérer (déployer) l'implant (20) au niveau du site,
dans lequel le composant d'extrémité distale cylindrique (4) inclut une première pluralité d'éléments de retenue d'implant (14), qui, pendant le retrait de la gaine (8) font saillie radialement vers l'extérieur depuis le composant d'extrémité distale cylindrique (4) pour venir en prise avec une surface orientée radialement vers l'intérieur de l'implant (20) pour empêcher l'implant (20) d'être emporté par la gaine (8) de manière proximale, par rapport au composant d'extrémité distale cylindrique (4), dans lequel la première pluralité d'éléments de retenue d'implant (14) sont formés à partir d'une partie de la paroi (6) du composant d'extrémité distale cylindrique (4), dans lequel la première pluralité d'éléments de retenue (14) sont configurés pour mettre en oeuvre un effet de mémoire de forme pour adopter une configuration en saillie radialement vers l'extérieur pour venir en prise avec la surface orientée radialement vers l'intérieur de l'implant (20) avant le retrait de la gaine (8), dans lequel au moins l'un de la première pluralité d'éléments de retenue d'implant (14) est une languette, dans lequel la languette comprend une partie charnière (16) au niveau de laquelle la languette est agencée pour fléchir, et une partie de mise en prise (19) agencée pour venir en prise avec l'implant (20), et dans lequel la partie charnière (16) s'étend le long d'une ligne de base et la partie de mise en prise (19) s'étend le long d'une ligne de contact pour entrer en contact avec l'implant (20), **caractérisé en ce que** : la languette est agencée pour s'étendre de manière distale depuis la ligne de base dans le composant d'extrémité distale cylindrique (4), et **en ce que** : la ligne de base de la partie charnière (16) est plus courte que la ligne de contact de la partie de mise en prise (19) en une direction circonférentielle.

2. Dispositif cathéter selon la revendication 1, dans lequel le composant d'extrémité distale cylindrique (4) inclut une seconde pluralité d'éléments de retenue d'implant (14), qui sont configurés pour mettre en oeuvre un effet de mémoire de forme pour faire saillie radialement vers l'extérieur du composant d'extrémité distale cylindrique (4) pour venir en prise avec une surface orientée radialement vers l'intérieur de l'implant (20) pour empêcher l'implant (20) d'être emporté de manière distale, par rapport au composant d'extrémité distale cylindrique (4).

3. Dispositif cathéter selon la revendication 2, dans lequel au moins l'un de la seconde pluralité d'éléments de retenue d'implant (14) est une languette agencée pour s'étendre de manière proximale depuis une ligne de base dans le composant d'extrémité distale cylindrique (4).

4. Dispositif cathéter selon une quelconque revendication précédente, dans lequel des pluralités d'au moins les première et seconde pluralités d'éléments de retenue d'implant (14) sont agencés sur une longueur du composant d'extrémité distale cylindrique (4), de sorte qu'une force exercée sur l'implant (20) par les au moins première et seconde pluralités d'éléments de retenue d'implant (14) soit distribuée uniformément sur une longueur de l'implant (20).

5. Dispositif cathéter selon la revendication 1, dans lequel la ligne de contact est jusqu'à deux fois plus longue que la ligne de base.

6. Dispositif cathéter selon une quelconque revendication précédente, dans lequel le composant d'extrémité distale cylindrique (4) est en nitinol.

7. Dispositif cathéter selon une quelconque revendication précédente, dans lequel le composant d'extrémité distale cylindrique (4) comprend au moins une section de retenue d'implant (30, 36) présentant au moins une parmi les première et/ou seconde pluralités d'éléments de retenue d'implant (14) et au moins une section flexible (32).

8. Dispositif cathéter selon une revendication 7, dans lequel au moins une section flexible (32) comprend une pluralité de fentes s'étendant circonférentiellement en quinconce (34) pour former une articulation cardanique.

9. Dispositif cathéter selon l'une quelconque des revendications 4 à 7 précédentes, dans lequel au moins une section de retenue d'implant (30, 36) et au moins une section flexible (32) sont alternées sur la longueur du composant d'extrémité distale cylindrique (4).

10. Dispositif cathéter selon l'une quelconque des revendications précédentes, dans lequel le composant d'extrémité distale cylindrique (4), dans lequel au moins la première pluralité d'éléments de retenue (14) et la seconde pluralité d'éléments de retenue (14) sont disposés dans la même section de retenue d'implant (30, 36).

11. Dispositif cathéter selon la revendication 10, dans lequel les première et seconde pluralités d'éléments de retenue (14) sont agencés circonférentiellement autour du composant d'extrémité distale cylindrique (4) en au moins une rangée, dans lequel les première et seconde pluralités d'éléments de retenue (14) sont alternés sur au moins une rangée.

12. Dispositif cathéter selon l'une quelconque des revendications précédentes, dans lequel la première pluralité d'éléments de retenue (14) sont disposés en un emplacement sur le composant d'extrémité distale cylindrique (4) qui est agencé pour être reçu par une région proximale de l'implant (20).

13. Dispositif cathéter selon l'une quelconque des revendications précédentes, dans lequel la seconde pluralité d'éléments de retenue (14) sont disposés en un emplacement sur le composant d'extrémité distale cylindrique qui est agencé pour être reçu par une région distale de l'implant (20).

14. Dispositif cathéter selon la revendication 12, dans lequel la languette s'étend en décrivant un arc sur la circonférence du composant d'extrémité distale cylindrique (4).

15. Dispositif cathéter selon l'une quelconque des revendications précédentes, dans lequel les première et/ou seconde pluralités d'éléments de retenue (14) adoptent une configuration dans laquelle les premiers et/ou seconds éléments de retenue sont fléchis au niveau de la partie charnière (16).

16. Dispositif cathéter selon une quelconque revendication précédente, dans lequel les première et/ou seconde pluralités d'éléments de retenue (14) sont configurés de sorte qu'une force exercée sur l'implant (20) par les première et/ou seconde pluralités d'éléments de retenue (14), respectivement, dans une plage de fléchissement de la partie charnière (16) soit sensiblement la même sur la plage de fléchissement.

17. Cathéter selon une quelconque revendication précédente, dans lequel la languette est configurée pour être au moins l'une parmi un crochet, un arc, un chevron ou une pique.

18. Cathéter selon une quelconque revendication précédente, dans lequel les première et/ou seconde pluralités d'éléments de retenue (14) sont non polis.

19. Dispositif cathéter selon une quelconque revendication précédente, dans lequel la première pluralité d'éléments de retenue d'implant (14) peuvent s'inscrire à l'intérieur de l'épaisseur de paroi.

20. Dispositif cathéter selon l'une quelconque des revendications précédentes, dans lequel la partie charnière (16) est sensiblement dans une région de palier de la courbe contrainte-déformation du matériau des au moins première et seconde pluralités d'éléments de retenue d'implant (14), lorsqu'ils sont en prise avec l'implant (20), de sorte que les au moins première et seconde pluralités d'éléments de retenue d'implant (14) exercent plus ou moins la même force sur l'implant (20) peu importe à quel point ils font saillie (fléchissent) depuis le composant d'extrémité distale cylindrique (4).

21. Dispositif cathéter selon l'une quelconque des revendications précédentes, dans lequel la ligne de base de la partie charnière (16) est plus courte que la ligne de contact de la partie de mise en prise (19) en une direction circonférentielle, de sorte que la force exercée sur un implant revêtu, revêtu d'un revêtement ePTFE, soit distribuée.

22. Procédé de fabrication d'un tube d'alliage à mémoire de forme pour un dispositif cathéter pour la pose par voie transluminale d'un implant tubulaire auto-déployable à un site dans un corps, le procédé comprenant : la formation d'une partie charnière (16) dans le tube d'alliage en mémoire de forme, la déformation du tube d'alliage à mémoire de forme par fléchissement d'une partie du tube d'alliage à mémoire de forme au niveau de la charnière, et le durcissement de la partie déformée par chauffage de l'alliage à mémoire de forme, de sorte que l'alliage à mémoire de forme, à température corporelle, puisse adopter une configuration dans laquelle la partie charnière (16) est fléchie radialement vers l'extérieur depuis le tube d'alliage à mémoire de forme, dans lequel la partie charnière (16) est comprise dans une languette (14), au niveau de laquelle partie charnière (16) la languette (14) est agencée pour fléchir, et une partie de mise en prise (19) agencée pour venir en prise avec l'implant, dans lequel la partie charnière (16) s'étend de manière distale le long d'une ligne de base et la partie de mise en prise (19) s'étend le long d'une ligne de contact pour entrer en contact avec l'implant (20), et dans lequel la ligne de base de la partie charnière (16) est plus courte que la ligne de contact de la partie de mise en prise (19) en une direction circonférentielle.

23. Procédé d'assemblage d'un ensemble cathéter pour la pose par voie transluminale d'un implant tubulaire auto-déployable à un site dans un corps, l'ensemble cathéter comprenant : un cathéter interne (2) comprenant un tube d'alliage à mémoire de forme, une gaine (8) et un implant auto-déployable (20), le procédé comprenant les étapes de : assemblage de l'implant auto-déployable sur le tube d'alliage à mémoire de forme, dans lequel à l'étape d'assemblage, le tube d'alliage à mémoire de forme est à une température qui lui permet de s'adapter, principalement grâce à un maclage martensitique, à la pression radialement vers l'intérieur qui lui est imposée par l'implant auto-déployable, dans lequel une languette (14) est fournie comprenant une partie charnière (16) au niveau de laquelle la languette est agencée pour fléchir, et une partie de mise en prise (19) agencée pour venir en prise avec l'implant (20),
dans lequel la partie charnière (16) s'étend le long d'une ligne de base et la partie de mise en prise (19) s'étend le long d'une ligne de contact pour entrer en contact avec l'implant (20), et dans lequel la ligne de base de la partie charnière (16) est plus courte que la ligne de contact de la partie de mise en prise (19) en une direction circonférentielle.
